(19) Europäisches Patentamt
European Patent Office
Office européen des brevets

(11) Publication number: **0 341 991 A2**

(12) # EUROPEAN PATENT APPLICATION

(21) Application number: **89304705.0**

(22) Date of filing: **10.05.89**

(51) Int. Cl.4: **C 07 D 501/59**
C 07 D 471/04,
A 61 K 31/545, A 61 K 31/435
//(C07D471/04,221:00,205:00)

(30) Priority: **13.05.88 US 193474**

(43) Date of publication of application:
**15.11.89 Bulletin 89/46**

(84) Designated Contracting States:
**AT BE CH DE ES FR GB GR IT LI NL SE**

(71) Applicant: **ELI LILLY AND COMPANY**
**Lilly Corporate Center**
**Indianapolis Indiana 46285 (US)**

(72) Inventor: **Engel, Gary Lowell**
**1984 Inverness Place**
**Greenwood Indiana 46143 (US)**

**Pfeiffer, Ralph Roberts**
**7021 North Central Avenue**
**Indianapolis Indiana 46220 (US)**

(74) Representative: **Tapping, Kenneth George et al**
**Erl Wood Manor**
**Windlesham Surrey, GU20 6PH (GB)**

(54) **Method for recovery of antibiotics from mother liquors and novel pharmaceutically-acceptable salts thereof.**

(57) Pharmaceutically-acceptable anthraquinone-1,5-disulfonate salts of selected cephalosporins and 1-carba(1-dethia)cephems are provided, as well as a process for preparing said salt forms.

FIG.1

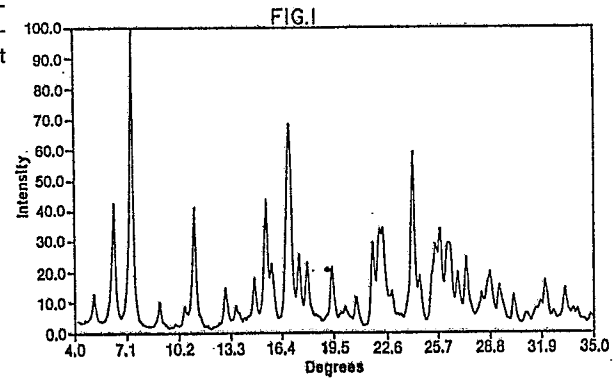


EP 0 341 991 A2

## Description

### METHOD FOR RECOVERY OF ANTIBIOTICS FROM MOTHER LIQUORS AND NOVEL PHARMACEUTICALLY-ACCEPTABLE SALTS THEREOF

This invention relates to a method for recovery of cephalosporin and 1-carba(dethia)cephalosporin antibiotics left over after crystallization of the final product. In particular, it relates to the recovery of the cephalosporin antibiotic cefaclor and certain 1-carba(dethia)cephalosporin antibiotics, for example, the 3-chloro-1-carba(dethia)cephalosporin, loracarbef. Cefaclor is claimed and described in U.S. Patent No. 3,925,372 and loracarbef in U.S. Patent No. 4,708,956. Both of these antibiotics are costly to manufacture and methods for their recovery during the final stages of manufacture can result in considerable savings. There are also provided novel pharmaceutically-acceptable salt forms of cephalosporin and 1-carba(dethia)cephalosporin antibiotics.

This invention provides a method for recovering from aqueous media the cephalosporin antibiotic cefaclor, the 3-chloro-1-carba(dethia)cephalosporin, loracarbef, the corresponding 3-methyl-1-carba(dethia)cephalosporin and substituted derivatives thereof which comprises mixing the mother liquors left over from the final-step crystallization of the product (typically a 1-2.5% solution) with anthraquinone-1,5-disulfonic acid disodium and adjusting the pH of the resulting suspension to 1 to 2, depending on the particular substrate, and allowing the pharmaceutically-acceptable antibiotic salt form to crystallize.

The resulting salt may then be used to treat bacterial infections in man and other animals. Alternatively, the salt may be treated with base and recrystallized to provide other crystalline forms, e.g., one pursued in the initial crystallization step. The anthraquinone-1,5-disulfonic acid disodium can also be recovered from an aqueous or non-aqueous medium (e.g., DMF) by titration with a caustic solution such as 1N NaOH.

The process of this invention is particularly useful in a tertiary recovery sense. Existing methodology teaches that some cephalosporins can be recovered from dilute solutions as crystalline solvates of dimethylformamide or acetonitrile. Additional recovery of the free antibiotic from the solvate form may require harsh conditions such as azeotropic distillation (typically used for solvent recovery) which, not surprisingly, leads to loss of product through decomposition. The salt form is significantly more stable than the zwitterionic form during the azeotrope process and additional recovery is possible. Thus, the present invention which provides recovery of the antibiotic as a highly crystalline pharmaceutically-acceptable salt, which may be used as is, or easily convertible to another salt form, stands as a significant advance.

Figure 1 shows an x-ray diffraction pattern (copper radiation, $\lambda$ = 1.540Å] for 7ß-D-phenylglycylamido-1-carba(1-dethia)-3-chloro-3-cephem-4-carboxylic acid anthraquinone-1,5-disulfonate salt. This compound is prepared as described in Example 2 below.

The present invention provides a process for crystallizing salts represented by Formula (I)

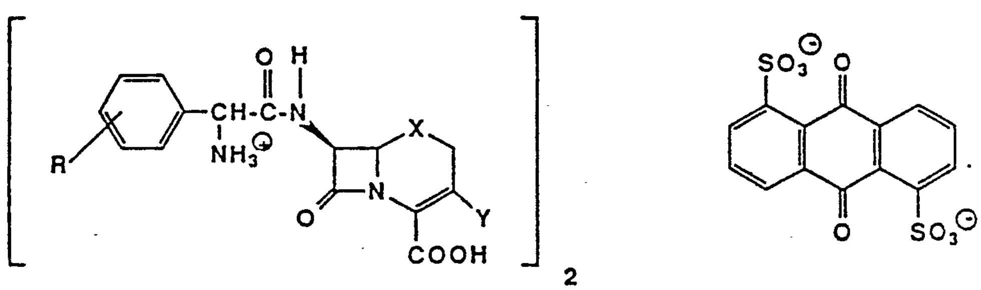

(I)

wherein X is sulfur or $-CH_2-$; Y is chloro; and R is hydrogen, hydroxy or ($C_1$-$C_4$ alkyl)-$SO_2NH$-; which comprises adding anthraquinone-1,5-disulfonic acid or an alkali metal salt thereof to a dilute aqueous solution of a compound of Formula (II):

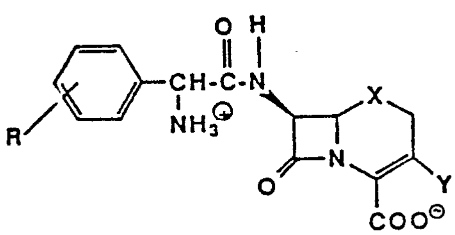

(II)

wherein X, Y and R have the above-defined meanings; and adjusting the pH to about 1 to about 2.

As used herein, the term $C_1$-$C_4$ alkyl denotes groups such as methyl, ethyl, n-propyl, isopropyl, n-butyl and isobutyl. Further, the term "dilute" refers to a solution of cephalosporin II at a concentration of about 0.5% to

about 2.5%. Finally, the term alkali metal salt refers to sodium or potassium.

The dilute aqueous solution of II used in the process can be the mother liquor of a crystallization of II or other spent aqueous solution of II such as a dilute reaction mixture or wash solution. The concentration of II in the aqueous medium is generally below about 3% by weight.

The acid employed in the process is preferably a mineral acid such as hydrochloric acid, sulfuric acid or phosphoric acid. The solubility of the anthraquinone salt I in the acidified aqueous media is such that dilute acids, e.g., 1N hydrochloric acid may be used or, if desired, even more dilute acid may be used.

Prior to acidification of the aqueous salt solution, insoluble impurities can be separated. Alternatively, the solution may be decolorized, e.g., by treatment with charcoal. Following acidification of the salt solution, the crystalline anthraquinone di-salt (I) is separated by conventional means, e.g., by filtration, centrifugation, decantation and the like. The salt may be further purified by washing with a suitable solvent such as acetone.

The process of the invention provides a method for recovering additional amounts of valuable antibiotics from spent process aqueous liquors. Generally, the zwitterionic forms of antibiotic (I) are soluble in such liquors at concentrations up to about 15 mg/ml depending upon the particular antibiotic. Yields of recovered antibiotic are generally about 80% to about 90% in the process.

The salt (I) obtained in the process can be converted to the parent antibiotic (II) in zwitterionic form by treatment of (I) with triethylamine in water, followed by acidification to the isoelectric point. The free antibiotic is then separated. Alternatively, I can be converted to the dimethylformamide solvate of II by treatment of a DMF solution of (I) with triethylamine. The DMF solvate of (II) is obtained crystalline and can be converted to a hydrate of (II).

As a further aspect of the present invention, there are provided pharmaceutically-acceptable salts of Formula (I)

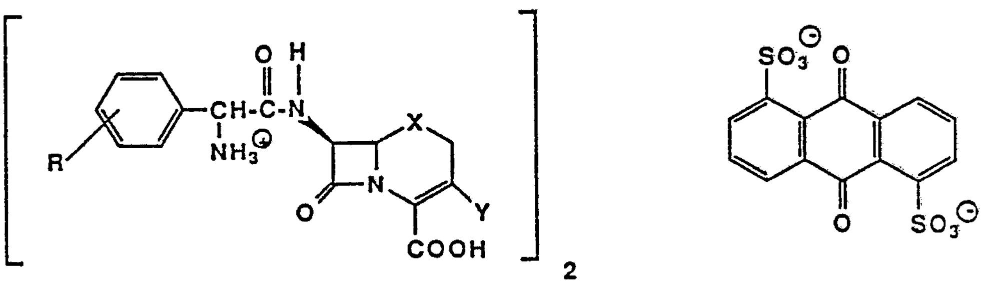

(I)

wherein X, Y and R have the above-defined meanings.

As yet a further aspect of this invention, there is provided a method for treatment of bacterial infection in man and other animals, which comprises administering to an infected host a therapeutically-effective amount of a compound of Formula (I), wherein X is sulfur or $-CH_2-$; y is chloro; and R is hydrogen, hydroxy or ($C_1$-$C_4$ alkyl)-$SO_2NH$-. A "therapeutically- effective amount" of the present pharmaceutically-acceptable salts may vary from about 250 mg to about 2 g per day.

While practicing the method of this invention for treating infectious diseases in man and other animals, the pharmaceutically-acceptable cephalosporin salt or 1-carba(dethia)cephalosporin salt can be administered as an antibiotic in a single daily dose or in multiple doses per day. The treatment regime may require administration over extended periods of time, e.g., for several days or for from two to three weeks. The amount administered per dose will depend on such factors as the nature and severity of the infection, the age and general health of the patient, the tolerance of both the patient and the microorganism or microorganisms involved in the infection to the antibiotic compound.

In the present invention, the following comprise the most preferred substrates of Formula (II):
7β-D-phenylglycylamido-1-carba(1-dethia)-3-chloro-3-cephem-4-carboxylic acid,
7β-D-(m-methylsulfonylamino)phenylglycylamido-1-carba(1-dethia)-3-chloro-3-cephem-4-carboxylic acid,
7β-D-(m-ethylsulfonylamino)phenylglycylamido-1-carba(1-dethia)-3-chloro-3-cephem-4-carboxylic acid,
7β-D-(p-hydroxy)phenylglycylamido-1-carba(1-dethia)-3-chloro-3-cephem-4-carboxylic acid, and
7β-D-phenylglycylamido-3-chloro-3-cephem-4-carboxylic acid.

Preferred reaction conditions for the above process include the utilization of 1.0 to 1.5 moles of anthraquinone disulfonate disodium salt, and a crystallization pH of about 1.5 to 1.8. After filtration, the crystals are filtered, washed with acetone and air dried.

In an example of the process of the present invention, an aqueous solution containing approximately 2% by weight of the desired substrate of Formula (II) is treated with an excess of anthraquinone-1,5-disulfonate disodium salt and the solution allowed to stir for about 0.5 h. The above solution is filtered to remove any undissolved materials and the resulting filtrate is treated with 1N HCl to achieve a pH of approximately 1.5 to 2.0, preferably about 1.5. After additional stirring, the anthraquinone-1,5-disulfonate salt of Formula (I) is separated by filtration, decantation or centrifugation and washed with acetone and air dried.

The following Examples are set forth to further describe the present invention but are in no sense to be construed as limiting the scope thereof.

## Experimental Section

### Example 1

7ß-D-Phenylglycylamido-3-chloro-3-cephem-4-carboxylic acid anthraquinone-1,5-disulfonate salt

A dilute solution of 7ß-D-phenylglycylamido-3-chloro-3-cephem-4-carboxylic acid is treated with 1 molar equivalent of anthraquinone-1,5-disulfonate disodium salt and acidified to a pH of about 1.8. The title compound crystallized from solution and is filtered, washed with acetone and air dried.

Empirical Formula: $C_{44}H_{36}N_6O_{16}S_4Cl_2 \bullet 5H_2O$

Molecular Weight : 1194.0

Elemental Analysis:

| Analysis | Theory | Found |
|---|---|---|
| C | 44.22% | 44.38% |
| H | 3.85 | 3.37 |
| N | 7.03 | 7.00 |
| O | 28.14 | 29.60 |

Solubility vs. pH:

| pH | Solubility |
|---|---|
| 1.80 | 0.6 mg/ml |
| 2.73 | 1.7 |
| 4.34 | 54.5 |

IR ($cm^{-1}$) (mull): 1035.4, 1086.8, 1127.0, 1169.4, 1188.8, 11.93.2, 1208.8, 1219,2, 1290.8, 1376.8, 1457.1, 1536.0, 1683.1, 1692.3, 1790.5, 2854.1.NMR (300 MHz, DMSO-$d_6$): δ: 3.5-3.9 (d of d, 2H); 5.0 (br s, 1H); 5.2 (d, 1H); 5.8 (d of d, 1H); 7.4-7.55 (m, 5H); 7.8 (t, 1H); 8.0 (d of d, 1H); 8.3 (d of d, 1H); 8.7 (br s, 3H); 9.8 (d, 1H).

### Example 2

7ß-D-Phenylglycylamido-1-carba(1-dethia)-3-chloro-3-cephem-4-carboxylic acid anthraquinone-1,5-disulfonate salt

In a manner substantially the same as that for Example 1, utilizing 7ß-D-phenylglycylamido-1-carba (1-dethia)-3-chloro-3-cephem-4-carboxylic acid as substrate, and while acidifying to a pH of about 1.5, the title compound was produced in about 50% yield.

Empirical Formula: $C_{46}H_{44}N_6O_{18}S_2Cl_2 \bullet 2H_2O$

Molecular Weight : 1103.9

Elemental Analysis:

| Analysis | Theory | Found |
|---|---|---|
| C | 50.00% | 50.32% |
| H | 3.99 | 4.03 |
| N | 7.61 | 7.46 |
| O | 26.09 | 24.95 |

Solubility vs. pH:

| pH | Solubility |
|---|---|
| 2.34 | 9.1 mg/ml |
| 2.75 | 17.3 |
| 3.06 | 31.2 |

IR ($cm^{-1}$) (mull): 1034.1, 11.81.3, 1213.8, 1219.5, 1377.0, 1456.6, 1461.0, 1537.1, 1680.7, 1683.7, 1730.5, 1783.5, 2854.2, 2869.9.NMR (300 MHz, DMSO-$d_6$): δ: 1.4 (m, 2H); 2.4-2.6 (m, 2H); 3.8 (m, 1H); 5.0 (br s, 1H); 5.4 (d of d,

1H); 7.4-7.5 (m, 5H); 7.7-7.8 (t, 1H); 8.0 (d of d, 1H); 8.3 (d of d, 1H); 8.6-8.8 (br s, 3H); 9.3 (d, 1H). See Figure 1 for the x-ray diffraction pattern.

## Example 3

7ß-D-(m-Methylsulfonylamino)phenylglycylamido-1-carba(1-dethia)-3-chloro-3-cephem-4-carboxylate anthroquinone-1,5-disulfonate salt

In a manner similar to that used in Example 1, utilizing 7ß-D-(m-methylsulfonylamino)phenylglycylamido-1-carba(1-dethia)-3-chloro-3-cephem-4-carboxylic acid as the starting material, while adjusting to a pH of about 1.8, the title compound was produced in about 65% yield.

## Example 4

The crystalline anthraquinone-1,5-disulfonate salts prepared in Examples 1 to 3 may be transformed back into the corresponding starting materials or solvates thereof by the following two methods:

a) Treatment of the anthraquinone-1,5-disulfonate salt of the cephalosporin (or 1-carba(1-dethia)-3-cephem) with triethylamine-$H_2O$, to the isoelectric pH provides the zwitterion or the hydrate of the cephalosporin (or 1-carba(1-dethia)-3-cephem); or

b) Treatment of the anthraquinone-1,5-disulfonate salt of the cephalosporin (or 1-carba(1-dethia)-3-cephem) with dimethylformamide, followed by triethylamine affords the dimethylformamide solvate of the corresponding cephalosporin (or 1-carba(1-dethia)-3-cephem).

**Claims**

1. A process for preparing a salt of the formula

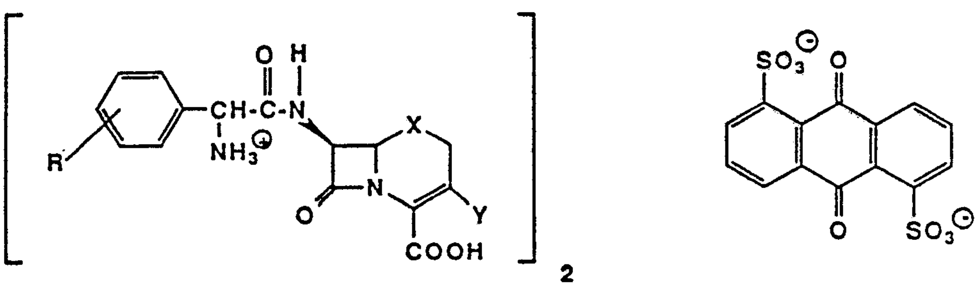

wherein X is sulfur or $-CH_2-$; Y is chloro; and R is hydrogen, hydroxy or ($C_1$-$C_4$ alkyl)-$SO_2$-NH-; which comprises adding anthraquinone-1,5-disulfonic acid or an alkali-metal salt thereof to a dilute aqueous solution of a compound of the formula

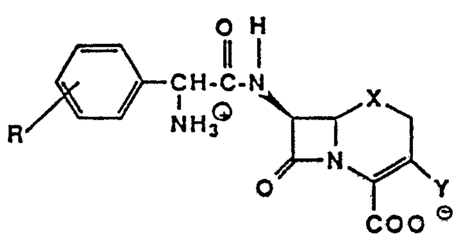

wherein X, Y and R have the above-defined meanings; and adjusting the pH to about 1.0 to about 2.5.

2. A process according to claim 1, wherein R is hydrogen.

3. A process according to claim 1, wherein R is ($C_1$-$C_4$ alkyl)-$SO_2$-NH-.

4. A compound of Formula (I):

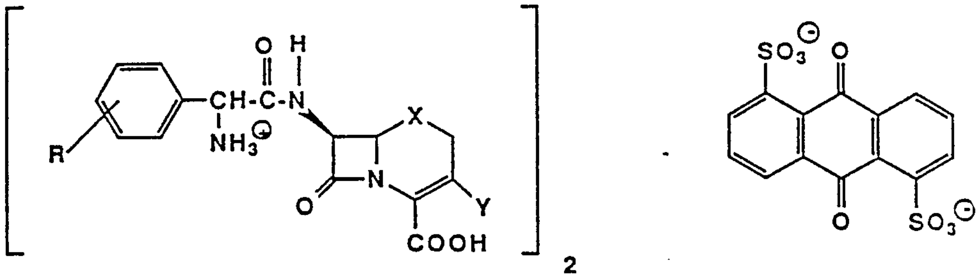

(I)

wherein X is sulfur or $-CH_2-$; Y is chloro; and R is hydrogen, hydroxy or $(C_1-C_4$ alkyl)$-SO_2-NH-$.

5. An anthraquinone-1,5-disulfonate salt of any one of the following compounds:
7ß-(D-phenylglycylamino)-3-chloro-1-carba(dethia)-3-cephem-4-carboxylic acid,
7ß-(D-phenylglycylamino)-3-chloro-3-cephem-4-carboxylic acid,
7ß-(D-p-hydroxyphenylglycylamino)-1-carba(1-dethia)-3-chloro-3-cephem-4-carboxylic acid,
7ß-(D-m-ethylsulfonylaminophenylglycylamino)-1-carba(1-dethia)-3-chloro-3-cephem-4-carboxylic acid, and
7ß-(D-m-methylsulfonylaminophenylglycylamino)-1-carba(1-dethia)-3-chloro-3-cephem-4-carboxylic acid.

6. A compound of Formula (I), as claimed in claim 4, for use as an antibacterial agent.

7. The anthraquinone-1,5-disulfoxide salt of any one of the following compounds:
7ß-(D-phenylglycylamino)-3-chloro-1-carba(dethia)-3-cephem-4-carboxylic acid,
7ß-(D-phenylglycylamino)-3-chloro-3-cephem-4-carboxylic acid,
7ß-(D-p-hydroxyphenylglycylamino)-1-carba(1-dethia)-3-chloro-3-cephem-4-carboxylic acid,
7ß-(D-m-ethylsulfonylaminophenylglycylamino)-1-carba(1-dethia)-3-chloro-3-cephem-4-carboxylic acid, and
7ß-(D-m-methylsulfonylaminophenylglycylamino)-1-carba(1-dethia)-3-chloro-3-cephem-4-carboxylic acid, for use as an antibacterial agent.

8. A pharmaceutical formulation comprising as an active ingredient a compound of Formula (I), as claimed in claim 4 or 5, associated with one or more pharmaceutically-acceptable carriers therefor.

**Claims for the following Contracting States: GR, ES**

1. A process for preparing a salt of the formula

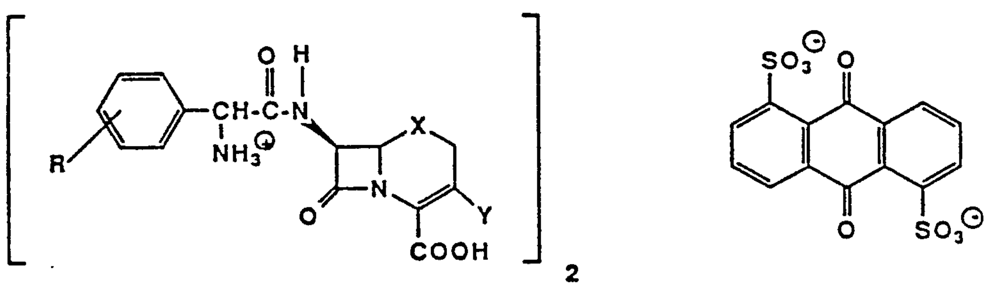

wherein X is sulfur or $-CH_2-$; Y is chloro; and R is hydrogen, hydroxy or $(C_1-C_4$ alkyl)$-SO_2-NH-$; which comprises adding anthraquinone-1,5-disulfonic acid or an alkali-metal salt thereof to a dilute aqueous solution of a compound of the formula

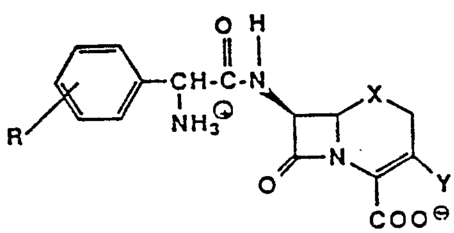

wherein X, Y and R have the above-defined meanings; and adjusting the pH to about 1.0 to about 2.5.

2. A process according to claim 1, wherein R is hydrogen.

3. A process according to claim 1, wherein R is $(C_1-C_4)$ alkyl)$-SO_2-NH-$.

4. A process according to claim 1, wherein R is hydroxy.

5. A process according to claim 3, wherein R is $CH_3SO_2-NH-$ and is in the meta position.

6. A process according to claim 3, wherein R is $CH_3CH_2-SO_2-NH-$ and is in the meta position.

7. A process according to any one of claims 1 to 6, wherein X is S.

8. A process according to any one of claims 1 to 6, wherein X is $-CH_2-$.

FIG.1

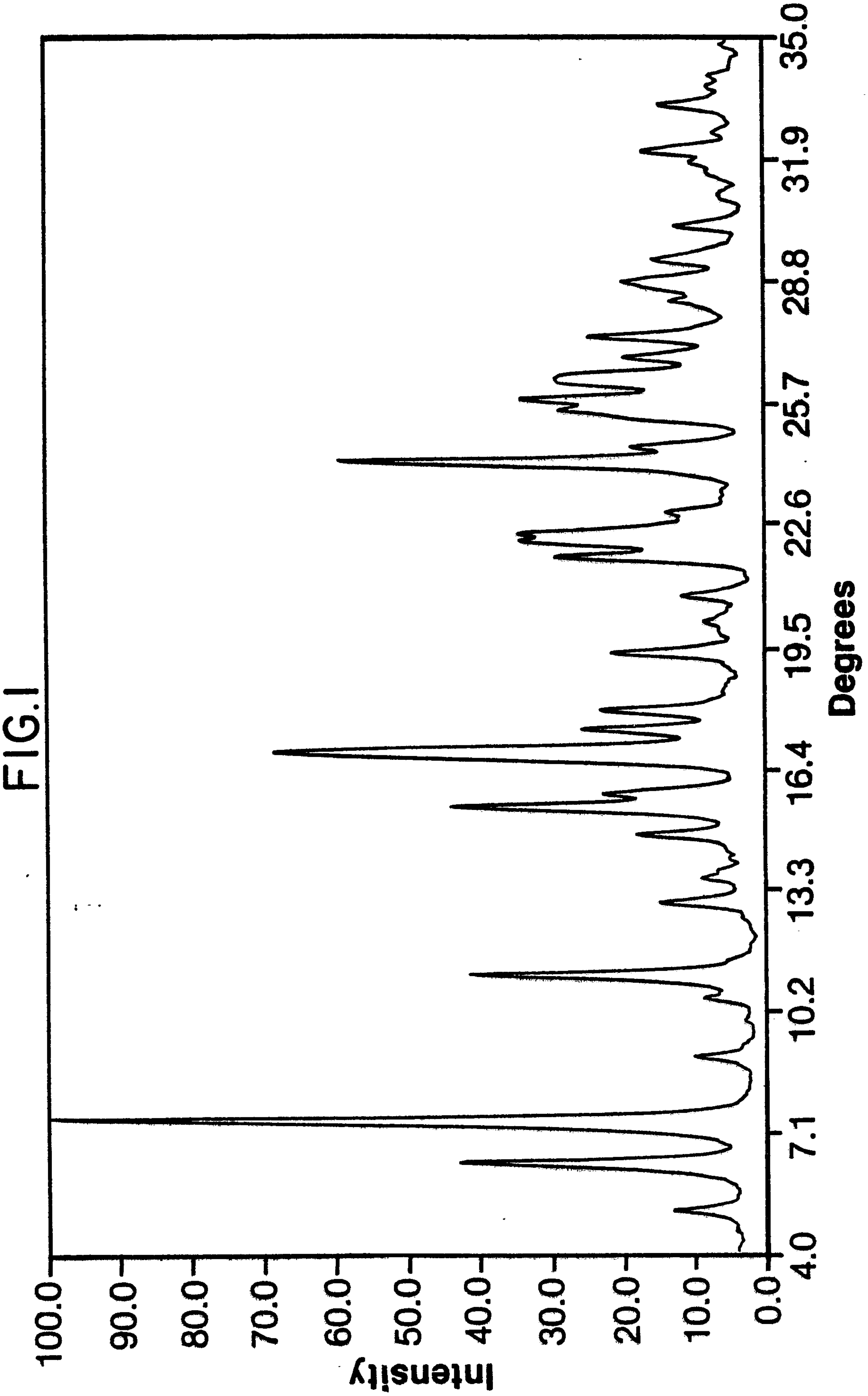
Intensity
100.0
90.0
80.0
70.0
60.0
50.0
40.0
30.0
20.0
10.0
0.0
4.0
7.1
10.2
13.3
16.4
19.5
22.6
25.7
28.8
31.9
35.0
Degrees